## Europäisches Patentamt

## European Patent Office

## Office européen des brevets

(11) Veröffentlichungsnummer: **0 407 400 B1**

# EUROPÄISCHE PATENTSCHRIFT

(45) Veröffentlichungstag der Patentschrift:
**27.11.91 Patentblatt 91/48**

(21) Anmeldenummer: **89902634.8**

(22) Anmeldetag: **18.01.89**

(86) Internationale Anmeldenummer:
**PCT/EP89/00054**

(87) Internationale Veröffentlichungsnummer:
**WO 89/06517 27.07.89 Gazette 89/16**

(51) Int. Cl.⁵: **A61B 17/22**

(54) **MEDIZINISCHER KATHETER MIT SCHNEIDVORRICHTUNG.**

(30) Priorität: **19.01.88 DE 3801318**

(43) Veröffentlichungstag der Anmeldung:
**16.01.91 Patentblatt 91/03**

(45) Bekanntmachung des Hinweises auf die
Patenterteilung:
**27.11.91 Patentblatt 91/48**

(84) Benannte Vertragsstaaten:
**DE FR GB IT**

(56) Entgegenhaltungen:
**EP-A- 0 086 048**

(56) Entgegenhaltungen:
**EP-A- 0 147 192**
**DE-A- 1 903 618**
**DE-A- 3 320 076**
**FR-A- 1 585 065**

(73) Patentinhaber: **STOCKSMEIER, Uwe**
**Hauptstrasse 23**
**W-8132 Tutzing (DE)**

(72) Erfinder: **STOCKSMEIER, Uwe**
**Hauptstrasse 23**
**W-8132 Tutzing (DE)**

(74) Vertreter: **Grättinger, Günter**
**Wittelsbacherstrasse 5 Postfach 16 49**
**W-8130 Starnberg (DE)**

**Beschreibung**

Die Erfindung betrifft einen medizinischen Katheter mit Schneidvorrichtung am vorderen Ende eines zum Einführen in Blutgefäße vorgesehenen Schlauches, wobei die Schneidvorrichtung ein rotierendes Schneidwerkzeug aufweist, dessen Rotationsdurchmesser kleiner ist als der Innendurchmesser des Schlauches und welches bezüglich des Schlauches in Richtung der Rotationsachse verschiebbar ist.

Ein derartiger Katheter ist aus der EP-A 0 086 048 bekannt. Die Schneidvorrichtung besteht hier aus einem Fräskopf und einer Antriebswelle, welche im Inneren des Schlauches verläuft, wobei der Fräskopf am vorderen Ende der Antriebswelle angeordnet ist und letztere von einem am anderen Ende angeordneten Motor in Rotation versetzt wird. Die Benützung des Katheters erfolgt in der Weise, daß zunächst der Schlauch in das Gefäß eingeführt und bis zu der zu beseitigenden Verengung geschoben wird, woraufhin anschließend das Schneidwerkzeug in den Schlauch eingeführt und durch diesen hindurchgeschoben wird, bis es am vorderen Ende desselben wieder austritt und zum Entfernen der Gefäßverengung eingesetzt werden kann.

Bei diesem bekannten Katheter ist es nachteilig, daß die Position des Schneidwerkzeuges bezogen auf das vordere Ende des Schlauches während der Anwendung des Katheters nicht eindeutig bestimmbar ist, unter anderem deshalb, weil sich bei gekrümmtem Katheter die Antriebswelle und der Schlauch im Bereich der Krümmung gegeneinander verschieben. Ein zu weit gegenüber den vorderen Ende des Schlauches vorgeschobenes Schneidwerkzeug kann jedoch unerwünschte Folgen verursachen, insbesondere das Gefäß verletzen.

Aus der DE-A 33 20 076 ist ferner ein Katheter bekannt, bei dem am vorderen Ende des Schlauches ein Schneidwerkzeug in Art einer Fräse vorgesehen ist, welches mittels einer damit gekoppelten Turbine antreibbar ist. Die Turbine wird mittels einer Druckflüssigkeit durch eine im Inneren des Schlauches verlaufende Druckleitung angetrieben. Bei Ausführung das Schlauches als Doppelschlauch kann gleichzeitig für einen Abtransport der Druckflüssigkeit und/oder des entfernten Gewebes gesorgt werden. Da bei diesem Katheter Schlauchende und Schneidwerkzeug nur gemeinsam vorgeschoben werden können, wird die Innenwand der Blutgefäße stark in Anspruch genommen; insbesondere bei weitgehend verschlossenen Blutgefäßen ist eine Gewebeverdrängung durch den Vorschub des Schlauchendes unvermeidbar.

Der vorliegenden Erfindung liegt die Aufgabe zugrunde, einen gattungsgemäßen Katheter so auszubilden, daß die Gefahr einer unerwünschten Beschädigung des Gewebes verringert wird.

Diese Aufgabe wird erfindungsgemäß dadurch gelöst, daß die Schneidvorrichtung oder nur das Schneidwerkzeug am vorderen Schlauchende gelagert ist, derart, daß das Schneidwerkzeug zwischen einer zurückgezogenen, durch einen ersten Anschlag definierten Ruhestellung, in welcher es vollständig im Inneren des Schlauches aufgenommen ist, und einer ausgefahrenen, durch einen zweiten Anschlag definierten Arbeitsstellung, in welcher es gegenüber dem Stirnende des Schlauches mehr oder weniger weit übersteht, verschiebbar ist.

Dabei besteht eine wesentliche Überlegung darin, daß das Schneidwerkzeug geringfügig über das vordere Schlauchende hinausgeschoben werden kann, so daß ein das Gefäß ganz oder teilweise verschließender Thrombus in sich zusammenfällt und das vordere Schlauchende unter Vermeidung von Quetschungen und Pressungen entsprechend der Abarbeitung des Thrombus weiter in das Blutgefäß hineingeschoben werden kann, das heißt, in dem Maße wie der Thrombus abgearbeitet wird, wird der Schlauch nachgefahren. Das Schneidwerkzeug ist trotzdem stets durch das vordere Schlauchende zentrisch gehalten, sodaß es die Gefäßwände nicht verletzen kann. Dadurch, daß die Arbeitsstellung des Schneidwerkzeuges durch einen Anschlag definiert ist, wird zuverlässig verhindert, daß das Gefäß durch ein zu weit vorstehendes Schneidwerkzeug in unerwünschter Weise beeinträchtigt wird. Nachdem bei dem erfindungsgemäßen Katheter keine Gefahr besteht, daß die Schneidvorrichtung zu weit aus dem Schlauch herausragt, ist die Entfernung der Gefäßverengung nicht nur weniger gefährlich als mit bekannten Kathetern, sondern kann darüberhinaus auch in kürzerer Zeit erfolgen, nachdem die Bedienung des Katheters eine geringere Präzision und ein weniger behutsames Vorgehen der Bedienungsperson toleriert, ohne daß dadurch das zu behandelnde Gefäß der Gefahr einer Verletzung ausgesetzt würde. Eine Verkürzung der Behandlungsdauer ist sowohl für den Patienten wie für den behandelnden Arzt ein großer Vorteil.

Die Sicherheit bei der Bedienung des Katheters wird noch verbessert durch das Merkmal gemäß Unteranspruch 3. Durch die starre Welle wird ein seitliches Ausweichen des als Schneidpropeller ausgebildeten Schneidwerkzeugs zuverlässig vermieden. Dadurch, daß der Schneidpropeller sich zentrisch in einen Thrombus einarbeitet, ist der erforderliche Freiraum für den Vorschub des Schlauchendes stets gewährleistet.

Diese Überlegung wird noch zusätzlich unterstützt durch das Merkmal von Unteranspruch 2, wonach schon durch die Ausbildung des Schneidpropellers dafür gesorgt ist, daß das Schneidgut zügig von der Schneidstelle in Richtung auf das Schlauchinnere abtransportiert wird. Diese Wirkung kann noch weiter verbessert werden durch Anschließen des Schlauches an eine Saugpumpe gemäß Unteranspruch 5. Bei einer bevorzugten Ausführungsform des erfindungsgemäßen Katheters ist der Überstand des Schneidwerkzeuges über das vordere Ende des Schlauches in der Arbeitsstellung einstellbar, wodurch eine optimale Anpaßbarkeit an den jeweiligen Einsatzfall möglich ist. Selbstverständlich kann das erfindungsgemäß vorgesehene Schneidwerkzeug auch in Kombination mit anderen zur Behandlung von Gefäßkrankheiten in die Gefäße einführbaren Geräten, z.B. Vorrichtungen zum Aufweiten von Gefäßen, verwendet werden.

Zum Antrieb des Schneidwerkzeuges kommen die aus dem Stand der Technik bekannten Möglichkeiten gleichermaßen in Frage. Der Antrieb kann somit über eine im Schlauchinneren angeordnete, flexible Antriebswelle erfolgen; die Schneidvorrichtung kann aber auch eine Turbine umfassen, welche mittels eines durch den Schlauch zugeführten Fluids angetrieben wird und mit dem Schneidwerkzeug drehfest verbunden ist. Bei der letztgenannten Ausführung kann in bekannter Weise der Schlauch als Doppelschlauch ausgebildet sein, wobei in der einen Kammer das dem Antrieb der Turbine dienende Fluid zugeführt wird, während es in der anderen Kammer abgeleitet wird. Die Verschiebung des Schneidwerkzeuges oder auch der gesamten Schneidvorrichtung kann in diesem Fall durch Veränderung der Druckverhältnisse in den beiden Kammern des Schlauches erfolgen.

Im folgenden werden zwei Ausführungsbeispiele der Erfindung anhand der Zeichnung erläutert. Es zeigen

> Fig. 1 einen schematischen Axialschnitt durch einen erfindungsgemäßen Katheter mit mechanischem Antrieb der Schneidvorrichtung und
> 
> Fig. 2 einen schematischen Axialschnitt durch einen erfindungsgemäßen Katheter mit hydraulischem Antrieb der Schneidvorrichtung.

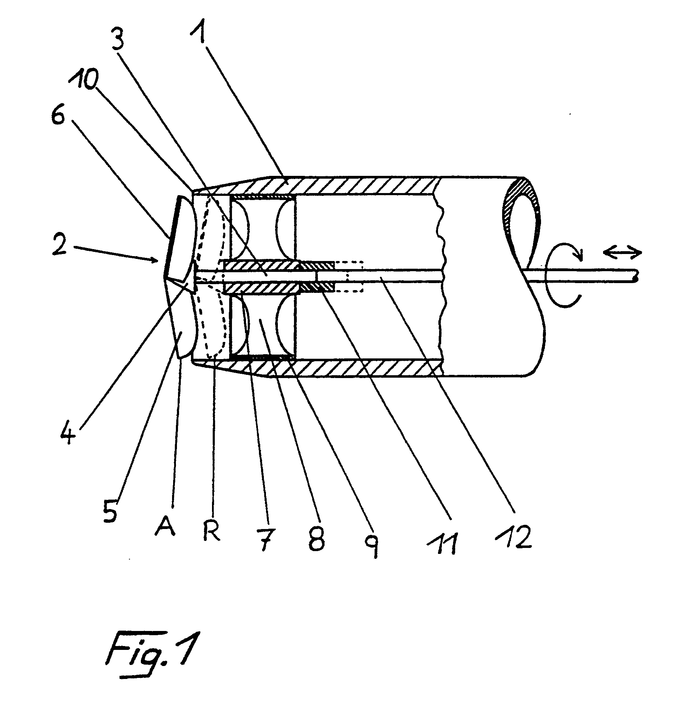

Fig. 1 zeigt in einem schematischen Axialschnitt durch das vordere Ende des Schlauches (1) eines medizinischen Katheters eine im Inneren des Schlauchendes angeordnete Schneidvorrichtung (2). Die Schneidvorrichtung (2) besteht aus einer zentrisch im Schlauch (1) drehbar gelagerten flexiblen Antriebswelle (12), die mit einer starren Welle (3) endet, an deren freiem Ende über eine Nabe (4) ein Schneidwerkzeug (5) angebracht ist. Das Schneidwerkzeug (5) trägt auf seiner dem Schlauch abgewandten Seite Schneidkanten (6), deren spezielle Form und Schneideigenschaften sich aus dem Verwendungszweck des erfindungsgemäßen Katheters ergeben. Die Welle (3) ist in einem Gleitlager (7) gelagert, das mittels Speichen (8) zentrisch in einer Buchse (9) gehalten wird. Die Buchse (9) ist fest in dem vorderen Ende des Schlauches (1), der zur Verringerung von Verletzungsgefahren für die Gefäße in seinem Endabschnitt leicht konisch verjüngt und an seinem Stirnende (10) abgerundet ist, angebracht.

In der Zeichnung ist das Schneidwerkzeug (5) in seiner Arbeitsstellung A dargestellt, in der es leicht über das Stirnende (10) des Schlauches (1) hinausragt. Die Position des Schneidwerkzeuges (5) in der Arbeitsstellung A ist dadurch vorgegeben, daß das dem Schneidwerkzeug abgewandte Ende des Gleitlagers (7) an einem die Verbindung der starren Welle (3) mit der flexiblen Antriebswelle (12) bildenden Anschlag (11) liegt.

Die gestrichelten Linien zeigen die Schneidvorrichtung in der Ruhestellung R, deren Position dadurch definiert ist, daß die Nabe (4) des Schneidwerkzeuges an dem ihr zugewandten Ende des Gleitlagers (7) anliegt.

Das Schneidwerkzeug (5) ist propellerförmig ausgebildet, so daß die mit Hilfe der Schneidkanten (6) vom Thrombus getrennte Substanz durch einen Sog in das Schlauchinnere befördert wird, aus dem sie zum Beispiel mittels einer nicht dargestellten Pumpe abgesaugt werden kann.

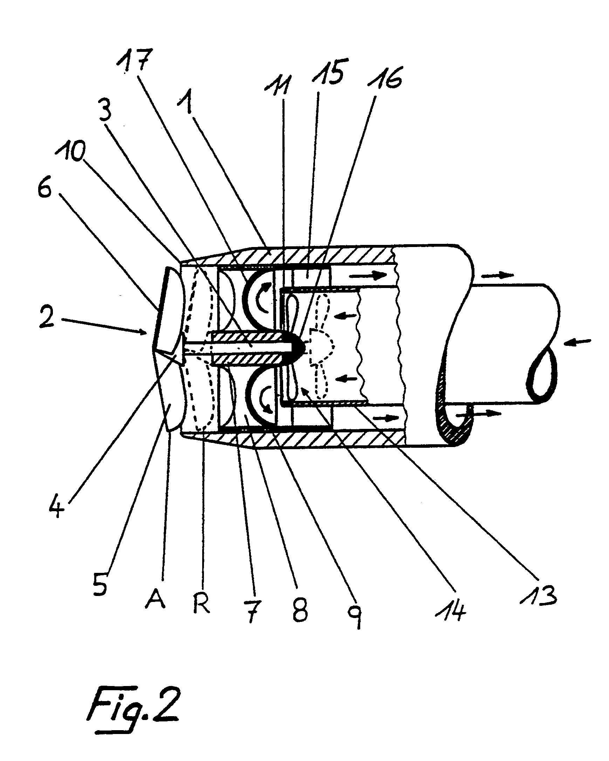

In Fig. 2 ist eine alternative Ausführungsform des erfindungsgemäßen Katheters dargestellt. Bei ihr erfolgt der Antrieb der Schneidvorrichtung mittels eines im Inneren des Schlauches zugeführten Druckmittels. Zu diesem Zweck ist in dem Schlauch (1) ein innerer Schlauch (13) angeordnet, in welchem das Druckmittel von einer (nicht dargestellten) Pumpe auf eine Turbine (14) geleitet wird, welche mit dem Schneidwerkzeug (5) über die starre Welle (3) drehfest verbunden ist. Das Ende des inneren Schlauches (13) ist mittels mehrerer Leitbleche (15) in der Buchse (9) zentrisch gehalten. Die Nabe (16) der Turbine (14) bildet den Anschlag (11), welcher die Arbeitsstellung (A) des Schneidwerkzeuges (5) definiert. Die Turbine ist so bemessen, daß sie die lichte Weite des inneren Schlauches (13) ausfüllt.

Zwischen der Buchse (9) und dem Gleitlager (7) ist ein Umlenkblech (17) angeordnet, welches das die Turbine (14) verlassende Druckmittel umlenkt und in den ringförmigen Hohlraum zwischen dem Schlauch (1) und dem inneren Schlauch (13) einleitet. Bei der dargestellten Strömungsrichtung, d.h. der Zuführung des Druckmittels durch den inneren Schlauch (13) wird die Turbine (14) mit ihrer Nabe (16) gegen das Gleitlager (7) gedrückt und hält dadurch das Schneidwerkzeug (5) in der Arbeitsstellung (A). Bei Umkehrung der Strömungsverhältnisse, d.h. Zuleitung des Druckmediums durch den ringförmigen Hohlraum zwischen dem Schlauch (1) und dem inneren Schlauch (13) wird die Turbine (14) durch das Druckmedium nach rechts bewegt und verschiebt dadurch das Schneidwerkzeug (5) in seine Ruhestellung (R), in der dessen Nabe (4) an dem Gleitlager (7) anliegt.

Der Überstand des Schneidwerkzeuges (5) über das vordere Ende des Schlauches (1) läßt sich beispielsweise dadurch einstellen, daß die Nabe (4) des Schneidwerkzeuges und/oder die starre Welle (3) gegen entsprechende Teile ausgetauscht werden, die eine größere oder kleinere axiale Erstreckung besitzen.

## Patentansprüche

1. Medizinischer Katheter mit Schneidvorrichtung (2) am vorderen Ende eines zum Einführen in Blutgefäße vorgesehenen Schlauches (1), wobei die Schneidvorrichtung (2) ein rotierendes Schneidwerkzeug (5) aufweist, dessen Rotationsdurchmesser kleiner ist als der Innendurchmesser des Schlauches (1), und welches bezüglich des Schlauches (1) in Richtung der Rotationsachse verschiebbar ist, dadurch gekennzeichnet, daß die Schneidvorrichtung (2) oder nur das Schneidwerkzeug (5) am vorderen Schlauchende gelagert ist, derart, daß das Schneidwerkzeug (5) zwischen einer zurückgezogenen, durch einen ersten Anschlag definierten Ruhestellung (R), in welcher es vollständig im Inneren des Schlauchendes aufgenommen ist, und einer ausgefahrenen, durch einen zweiten Anschlag definierten Arbeitsstellung (A), in welcher es gegenüber dem Stirnende des Schlauches (1) mehr oder weniger weit übersteht, verschiebbar ist.

2. Katheter nach Anspruch 1, dadurch gekennzeichnet, daß das Schneidwerkzeug (5) in Art eines Schneidpropellers ausgebildet ist, dessen Schaufeln Schneidkanten (6) aufweisen und zum Erzeugen eines in Richtung auf das Schlauchende gerichteten Soges geeignet ausgebildet sind.

3. Katheter nach Anspruch 1 oder 2, dadurch gekennzeichnet, daß das Schneidwerkzeug (5) über eine im Schlauchinneren verlaufende flexible Antriebswelle (12) angetrieben ist und daß ein kurzer an das Schneidwerkzeug anschließender Abschnitt der Antriebswelle zum Zwecke der längsverschieblichen Lagerung des Schneidwerkzeugs (5) als starre Welle (3) ausgebildet ist.

4. Katheter nach Anspruch 3, dadurch gekennzeichnet, daß das Schneidwerkzeug (5) fliegend am Ende der starren Welle (3) befestigt ist, welche in einem Gleitlager gelagert ist, das im Zentrum einer das Schlauchende von innen auskleidenden Buchse (9) angeordnet und mit dieser über wenige Speichen (8) verbunden ist.

5. Katheter nach Anspruch 1, gekennzeichnet dadurch , daß der Schlauch (1) als Saugschlauch ausgebildet und an eine Saugpumpe anschließbar ist.

6. Katheter nach Anspruch 1, dadurch gekennzeichnet, daß der Überstand des Schneidwerkzeuges (5) über das vordere Ende des Schlauches (1) in der Arbeitsstellung (A) einstellbar ist.

7. Katheter nach Anspruch 1 oder 2, dadurch gekennzeichnet, daß die Schneidvorrichtung (2) eine Turbine umfaßt, welche mittels eines durch den Schlauch (1) zugeführten Druckmittel angetrieben wird und mit dem Schneidwerkzeug (5) drehfest verbunden ist.

8. Katheter nach Anspruch 7, dadurch gekennzeichnet, daß die gesamte Schneidvorrichtung (2) bezüglich des Schlauches (1) axial verschiebbar ist.

9. Katheter nach Anspruch 8, dadurch gekennzeichnet, daß der Schlauch zwei Kammern enthält, von denen eine der Zuführung und die andere der Rückführung des Druckmittels dient, und daß die Verschiebung der Schneidvorrichtung dadurch erfolgt, daß die Strömungsverhältnisse des Druckmittels umgekehrt werden.

## Claims

1. Medical catheter with cutting device (2) provided at the front end of a tube (1) for introducing into blood vessels, the cutting device (2) comprising a rotating cutting tool (5), the diameter of rotation of which is smaller than the inner diameter of the tube (1) and which can be shifted along the axis of rotation relative to the tube (1), characterized in that the cutting device (2) or only the cutting tool (5) is positioned at the front end of the tube in such a way that the cutting tool (5) can be shifted between a retracted rest position (R) defined by a first stop, in which said tool is completely inserted within the end of the tube, and an extended working position (A) defined by a second stop, in which said tool protrudes more or less from the front end of the tube (1).

2. Catheter according to claim 1, characterized in that the cutting tool (5) is a sort of cutting propeller, the blades of which comprise cutting edges (6) and are able to build up a suction towards the end of the tube.

3. Catheter according to claim 1 or 2, characterized in that the cutting tool (5) is driven by way of a flexible power shaft (12) arranged within the tube and that a short portion of the power shaft adjacent to the cutting tool is formed as a rigid shaft (3) so as to provide for longitudinal shifting,of the cutting tool (5).

4. Catheter according to claim 3, characterized in that the cutting tool (5) is over-mounted at the end of the rigid shaft (3), said shaft running on a sliding bearing which is arranged at the center of a bush (9) lining the inside of the tube end, said bearing being connected with said bush by few spokes (8).

5. Catheter according to claim 1, characterized in that the tube (1) is designed as a suction tube being connectable to a suction pump.

6. Catheter according to claim 1, characterized in that the portion of the cutting tool (5) protruding beyond the front end of the tube (1) in its working position (A) is adjustable.

7. Catheter according to claim 1 or 2, characterized in that the cutting device (2) comprises a turbine driven by a pressurizing medium introduced through the tube (1) and fixedly connected with the cutting tool (5).

8. Catheter according to claim 7, characterized in that the whole cutting device (2) is shiftable in direction of the axis of the tube (1).

9. Catheter according to claim 8, characterized in that the tube comprises two chambers, one of them applying the pressurizing medium, the other for the backflow of said medium, whereby shifting movement of the cutting device occurs when the pressure flow of the pressurizing medium is reversed.

## Revendications

1. Cathéter médical à dispositif de coupe (2) se trouvant à l'extrémité antérieure d'un tuyau (1) destiné à être introduit dans des vaisseaux sanguins, le dit dispositif de coupe (2) comprenant un outil tranchant rotatif (5) dont le diamètre de rotation est plus petit que le diamètre intérieur du tuyau (1) et le dit outil tranchant étant déplaçable par rapport au tuyau (1) en direction de l'axe de rotation, caractérisé en ce que le dispositif de coupe (2) ou seulement l'outil tranchant (5) est logé à l'extrémité antérieure du tuyau, de telle sorte que l'outil tranchant (5) peut être déplacé entre une position de repos (R) rétractée, définie par un premier arrêt, position dans laquelle l'outil est totalement logé à l'intérieur du bout du tuyau, et une position de travail (A) avancée, définie par un second arrêt, position dans laquelle l'outil dépasse plus ou moins la tête du tuyau (1).

2. Cathéter selon la revendication 1, caractérisé en ce que l'outil tranchant (5) est formé de la façon d'une hélice tranchante dont les pales comprennent des lames tranchantes (6) pour produire une sucion en direction du bout du tuyau.

3. Catéther selon la revendication 1 ou 2, caractérisé en ce que l'outil tranchant (5) est actionné par un arbre de transmission (12) flexible positionné à l'intérieur du tuyau et qu'une courte portion de l'arbre de transmission adjacente à l'outil tranchant est constituée par un arbre fixe (3) pour le déplacement longitudinal de l'outil tranchant (5).

4. Cathéter selon la revendication 3, caractérisé en ce que l'outil tranchant (5) est fixé en porte-à-faux à l'extrémité de l'arbre fixe (3), qui est logé dans un palier-glisseur disposé au centre d'une douille (9) revêtant l'intérieur du bout du tuyau, et relié à celle-ci par quelques rayons (8).

5. Cathéter selon la revendication 1, caractérisé en ce que le tuyau (1) est constitué par un tuyau suceur et qu'il est reliable à une pompe aspirante.

6. Cathéter selon la revendication 1 , caractérisé en ce que la portion de l'outil tranchant (5) dépassant le bout antérieur du tuyau (1) dans la position de travail (A) est réglable.

7. Cathéter selon la revendication 1 ou 2, caractérisé en ce que l'outil tranchant (2) comprend une turbine actionnée par un moyen de pression introduit à travers le tuyau (1) et fixée conrotativement à l'outil tranchant (5).

8. Cathéter selon la revendication 7, caractérisé en ce que l'outil tranchant (2) est dans sa totalité déplaçable axialement par rapport au tuyau (1).

9. Cathéter selon la revendication 8, caractérisé en ce que le tuyau contient deux chambres dont l'une sert à l'introduction et l'autre à la restitution du moyen de pression, et que le déplaçage de l'outil tranchant résulte de ce que le rapport de circulation du moyen de pression est inversé.

*Fig.1*

*Fig.2*